# EUROPEAN PATENT APPLICATION

(11) **EP 1 666 882 A2**
(43) Date of publication of application: **07.06.2006**
(21) Application number: 05010740.8
(22) Date of filing: 18.05.2005
(51) Int. Cl.: G01N 33/68, G01N 33/58, G01N 33/533

(54) **Method for detecting protein as fluorescence image**

(30) Priority: 18.05.2004 JP 2004147824
(71) Applicant: FUJI PHOTO FILM CO., LTD., Minami-ashigara-shi, Kanagawa 250-0193 (JP)
(72) Inventor: Miura, Kenji Fuji Photo Film Co., Ltd., Tokyo 106-8620 (JP)
(74) Representative: Albrecht, Thomas

(57) **Abstract**

It is an object of the present invention to provide a method for enabling the detection of a trace amount of protein contained in an electrophoresis gel, which cannot visually be observed even in CBB staining capable of confirming a protein by visual observation, so as to enable the combined use of visual observation when a sample is cut off, thereby improving the reliability of analyses using a mass spectroscope or the like. The present invention provides a method for detecting a protein as a fluorescence image, which comprises staining a protein that has been electrophoresed on a gel with Coomassie Brilliant Blue and then applying excitation light having a wavelength of 600 nm or more to the protein.

## Description

### TECHNICAL FIELD

The present invention relates to a method for analyzing a protein, and specifically to a method for detecting a protein as a fluorescence image. More specifically, the present invention relates to a method for detecting a protein as a fluorescence image, which comprises applying a light having a certain excitation wavelength to a gel containing a protein that has been electrophoresed and then stained with Coomassie Brilliant Blue.

### BACKGROUND ART

Polyacrylamide gel electrophoresis (PAGE) has widely been used for separation, detection, and identification of proteins. PAGE is also useful in determining the size of a protein. In particular, in the case of SDS-PAGE (Sodium Dodecyl Sulfate-PAGE), a protein binds to SDS, which is an anionic surfactant, and thus the surface thereof becomes negatively charged. This method is characterized in that only the size of a protein molecule acts as a separation factor. Since SDS-PAGE has easy operatability and superior separating ability, it has widely been used as a technique of analyzing proteins. Since proteins are generally colorless when they are subjected to electrophoresis, an appropriate detection means is required. Known protein detection methods include silver staining, organic dye staining, and fluorescent staining.

Silver staining is a method comprising allowing silver ion to deposit in a gel and then reducing it. It exhibits the highest sensitivity among nonradioactive detection methods. However, this method is problematic in that it is relatively difficult to handle, and that it requires multiple steps.

Organic dye staining is a method for staining a protein band with organic dyes such as Amido Black 10B, Ponceaus S, Fast Green FCF, or Coomassie Brilliant Blue (CBB). In particular, Coomassie Brilliant Blue (CBB) staining has widely been used because a protein can be detected by visual observation after electrophoresis. For example, Jens Wiltfang et al., Electrophoresis 1991, 12, 352-366 describes that a protein or peptide can be detected by Coomassie Brilliant Blue (CBB) staining using an SDS-PAGE buffer solution. In particular, in the field of proteomics, in which a sample is cut out of gel after gel electrophoresis and analyzed with a MALDI-TOF mass spectroscope, CBB staining is considered to be a superior staining method because it does not impair mass spectrometry. However, such CBB staining is disadvantageous in terms of low detection sensitivity. Thus, a small amount of protein cannot generally be detected by CBB staining.

In order to develop a protein detection method that has a higher sensitivity than that of CBB staining, various types of fluorescent staining methods have been developed. Fluorescent staining is a method for detecting a protein that is an analysis target by labeling it with fluorescent dye, and it shows high sensitivity. For example, the following method has been known. After a protein has been subjected to electrophoresis, the protein is stained with a fluorescent dye used for staining proteins, such as SYPRO Orange or SYPRO Red manufactured by Molecular Probes, which has a peak excitation wavelength at 440 to 500 nm and a peak fluorescence wavelength at 560 to 620 nm, and acts mainly on a sodium dodecyl sulfate (SDS)-protein complex, so as to stain it, or with another fluorescent dye for staining proteins, which has a peak excitation wavelength at 510 to 580 nm and a peak fluorescence wavelength at 600 to 660 nm. Thereafter, excitation light is applied to the protein, so as to photoelectrically detect generated fluorescence. Thus, a fluorescence image of the protein is generated, thereby detecting positional information regarding the protein. However, since the aforementioned fluorescent reagent is transparent, this method is problematic in that the target protein cannot be confirmed by visual observation.

### DISCLOSURE OF THE INVENTION

It is an object of the present invention to solve the aforementioned problems of the prior art techniques. In other words, it is an object of the present invention to provide a method for enabling the detection of a trace amount of protein contained in an electrophoresis gel, which cannot visually be observed even in CBB staining capable of confirming a protein by visual observation, so as to enable the combined use of visual observation when a sample is cut off, thereby improving the reliability of analyses using a mass spectroscope or the like.

As a result of intensive studies directed towards achieving the aforementioned object, the present inventors have found that even a trace amount of protein can be detected as a fluorescence image by staining a protein that has been electrophoresed on a gel with Coomassie Brilliant Blue, applying an excitation light having a wavelength of 600 nm or more to such a protein, and detecting the emitted fluorescence, thereby completing the present invention.

That is to say, the present invention provides a method for detecting a protein as a fluorescence image, which comprises staining a protein that has been electrophoresed on a gel with Coomassie Brilliant Blue and then applying excitation light having a wavelength of 600 nm or more to the protein.

Preferably, excitation light having a wavelength of 600 nm or more is applied to a protein, and the generated fluorescence is photoelectrically detected, so as to generate a fluorescence image of the above protein.

Preferably, excitation light having a wavelength between 600 nm and 700 nm can be applied to the protein. More preferably, excitation light having a wavelength between 650 nm and 700 nm can be applied to the protein.

Specific examples of a gel to be used herein may include an agarose gel, an isoelectric focusing gel, and a polyacrylamide gel. Of these, the polyacrylamide gel is preferable.

Preferably, after a protein has been stained with Coomassie Brilliant Blue, it is decolorized with a decolorizing solution, and excitation light having a wavelength of 600 nm or more can be then applied to the resultant protein.

Preferably, a protein existing on a gel is detected by visual observation, and at the same time, it can also be detected as a fluorescence image.

### BRIEF DESCRIPTION OF THE DRAWINGS

Figure 1 shows a digitized image.
Figure 2 shows a fluorescence image (comparative example) excited at 532 nm.
Figure 3 shows a fluorescence image (the present invention) excited at 635 nm.
Figure 4 shows a fluorescence image (the present invention) excited at 670 nm.

### BEST MODE FOR CARRYING OUT THE INVENTION

The embodiments of the present invention will be described in detail below.

### (1) Preparation of protein solution

Any given protein can be electrophoresed in the present invention. When a protein is subjected to electrophoresis, it is preferable that a solution containing the protein be prepared, and that this solution be applied to a gel. A common buffer that has been known to persons skilled in the art can be used as a buffer for preparing a protein solution. For example, a buffer containing Tris-HCl, SDS, β-mercaptoethanol, glycerol, or the like can be used. In addition, an indicator (e.g. bromphenol blue, etc.) may also be added to such a buffer, so as to confirm the progress of electrophoresis.

A protein contained in a protein solution may be any one of a natural protein derived from organisms, a chemically synthesized protein, and a recombinant protein produced by gene recombination. Moreover, it may be either a known protein or an unknown protein. A single type of protein or two or more types of proteins may be contained in a protein solution. Otherwise, a liquid containing components other than proteins, such as a body fluid derived from organisms, a treated product thereof, or a cell culture product, may also be used as a protein solution.

### (2) Electrophoresis

Electrophoresis of a protein can be carried out by methods known to persons skilled in the art. The type of gel used herein is not particularly limited. The gel is preferably a polyacrylamide gel, isoelectric focusing gel, or agarose gel, and particularly preferably a polyacrylamide gel. The concentration of polyacrylamide is not particularly limited, and it may appropriately be determined, taking into consideration the molecular weight of the protein to be electrophoresed, or the like. It is generally between approximately 3% and 25%, and preferably between approximately 3% and 20%. In addition, the concentration of polyacrylamide may be uniform throughout the gel as a whole, or may be gradated. When a gradient is established, a polyacrylamide gel having a concentration gradient such as 3% to 25% or 3% to 20% can be used. A commonly used buffer solution can be used as a buffer solution used for electrophoresis. For example, a buffer solution containing Tris, glycine, and SDS can be used. Electrophoresis can be carried out with an appropriate electric current for a certain period of time.

### (3) Staining and decolorization

The electrophoresed gel can be stained by immersion in a staining solution containing Coomassie Brilliant Blue for a certain period of time. During staining, the gel may be shaken. As Coomassie Brilliant Blue used in the present invention, either Coomassie Brilliant Blue R or Coomassie Brilliant Blue G may be used. As a staining solution, an aqueous solution containing 1% to 5% (w/v) Coomassie Brilliant Blue, 1% to 70% (v/v) methanol or ethanol, and 1% to 10% (v/v) acetic acid can be used, for example. The period of time required for allowing the gel to come into contact with the staining solution is not particularly limited. It may be, for example, 1 minute to 10 hours, preferably 10 minutes to 10 hours, and more preferably 30 minutes to 2 hours.

The stained gel can be decolorized with a decolorizing solution, as desired. As such a decolorizing solution, an aqueous solution containing 1% to 20% (v/v) methanol or ethanol and 1% to 20% (v/v) acetic acid can be used, for example. Decolorization can be carried out while exchanging the decolorizing solution with a fresh solution at times. The period of time required for allowing the gel to come into contact with the decolorizing solution is not particularly limited. It may be 1 minute or more, for example. Decolorization may also be carried out for approximately 1 day.

### (4) Image analysis

Fluorescence detection of a protein stained with Coomassie Brilliant Blue can be carried out using a standard fluorescent scanner such as a common image analyzer. A fluorescent image of a protein stained with Coomassie Brilliant Blue can be obtained, for example, using an FLA-5100 image analyzer that is commercially available from Fuji Photo Film Co., Ltd.

In this case, excitation light is applied to the stained protein by application means such as laser light source. The wavelength of such excitation light is preferably between 600 nm and 700 nm. It may be 635 nm or 670 nm, for example. The wavelength of excitation light is more preferably between 650 nm and 700 nm. It may be 670 nm, for example. The above-described application means is preferably a laser having a radiation beam with a wavelength of 600 nm or more. For example, a laser beam generated from an He-Ne gas laser, or a diode laser that is in a solid state, can be applied to the protein, for example. Subsequently, fluorescence is detected with a photosensitive detector (e.g. a photomultiplier tube, an electrically charged binding device, etc.).

A photosensitive detector is composed of an optical filter for selectively transmitting a wavelength of interest and a photoelectric transducer. It detects fluorescence from a protein and converts it into an electrical signal. A fluorescence pattern that is converted into an electrical signal indicates an electrophoretic pattern of a protein separated by electrophoresis.

The present invention will be further specifically described in the following examples. However, the examples are not intended to limit the present invention.

### EXAMPLES

### (1) Preparation of protein solution

Using a sample buffer having the composition shown in the following Table 1, a protein solution containing all proteins shown in the following Table 2 was prepared.

**Table 1:**

| Sample buffer | |
|---|---|
| 0.5 M Tris-HCl (pH 6.8) | 1 ml |
| 10% SDS | 2 ml |
| β-mercaptoethanol | 0.6 ml |
| Glycerol | 1 ml |
| Purified water | 5.4 ml |
| 1% bromphenol blue | Several droplets |
| Total | 10 ml |

**Table 2:**

| Types and concentrations of proteins | | |
|---|---|---|
| Protein | Molecular weight (kDa) | Concentration (µg/1000 µl) |
| Myosin | 205 | 56 |
| β-galactosidase | 116 | 39 |
| Phosphorylase b | 97.4 | 47 |
| Bovine serum albumin | 69 | 51 |
| Glutamate dehydrogenase | 55 | 206 |
| Lactate dehydrogenase | 36.5 | 62 |
| Carbonic anhydrase | 29 | 77 |
| Trypsin inhibitor | 20.1 | 84 |
| Lysozyme | 14.3 | 80 |
| Aprotinin | 6.5 | 92 |
| Insulin | 3.5 | 206 |

### (2) Electrophoresis

The protein solution prepared in (1) above was applied to each lane of a polyacrylamide gel (10 x 10 cm) with a concentration of 10%, at amounts of 5 µl (lanes 1 and 2), 2.5 µl (lanes 3 and 4), 1.25 µl (lanes 5 and 6), 0.625 µl (lanes 7 and 8), and 0.313 µl (lanes 9 and 10). Using an electrophoresis buffer (15.15 g of Tris, 72.05 g of glycine, 5 L of purified water, and 5 g of SDS), electrophoresis was carried out at 40 mA (constant current) for approximately 60 minutes, until the edge of bromphenol blue contained in the sample buffer reached the lower end of the gel.

### (3) Staining and decolorization

After completion of the electrophoresis, the gel was immersed in a staining solution (25 g of Coomassie Brilliant Blue R250, 500 ml of methanol, 50 ml of acetic acid, and 450 ml of purified water), and it was then shaken at a slow rate for 1 hour, so as to stain it. After completion of the staining, decolorization was carried out overnight, while exchanging the decolorizing solution (50 ml of methanol, 70 ml of acetic acid, and 880 ml of purified water) with a fresh solution at times.

### (4) Image analysis

Using an FLA-5100 image analyzer that is commercially available from Fuji Photo Film Co., Ltd., various types of images of gel stained with CBB were obtained under the following conditions.

**Table 3:**

| Conditions for image analysis | | |
|---|---|---|
| Conditions determined | Digitized image | Fluorescence images |
| Mode | Digitize | 1 laser, 1 image |
| Excitation wavelength | 532 nm | 532 nm, 635 nm, 670 nm |
| Filter | LPG filter | LPG, LPR, LPFR filters |
| PMT voltage | 250 HV | 600 HV |
| Pixel size | 200 microns | 200 microns |
| PMT used | Channel 1 | Channel 1 was used for excitation at 532 nm and at 635 nm. Channel 2 was used for excitation at 670 nm. |

LPG: Long Pass Green, which gives passage to 575 nm or more.
LPR: Long Pass Red, which gives passage to 665 nm or more.
LPFR: Long Pass Far Red, which gives passage to 705 nm or more.
HV: High Voltage, which indicates the voltage applied to the photomultiplier tube(PMT)

### (5) Printout of image

The image obtained using FLA-5100 was opened within MultiGauge software of ScienceLab, which is commercially available from Fuji Photo Film Co., Ltd. Contrast control was displayed by the same method (exponential conversion), such that it was possible to carry out comparison of the images, following which the resulting images were printed out.

### (6) Explanation of printed image

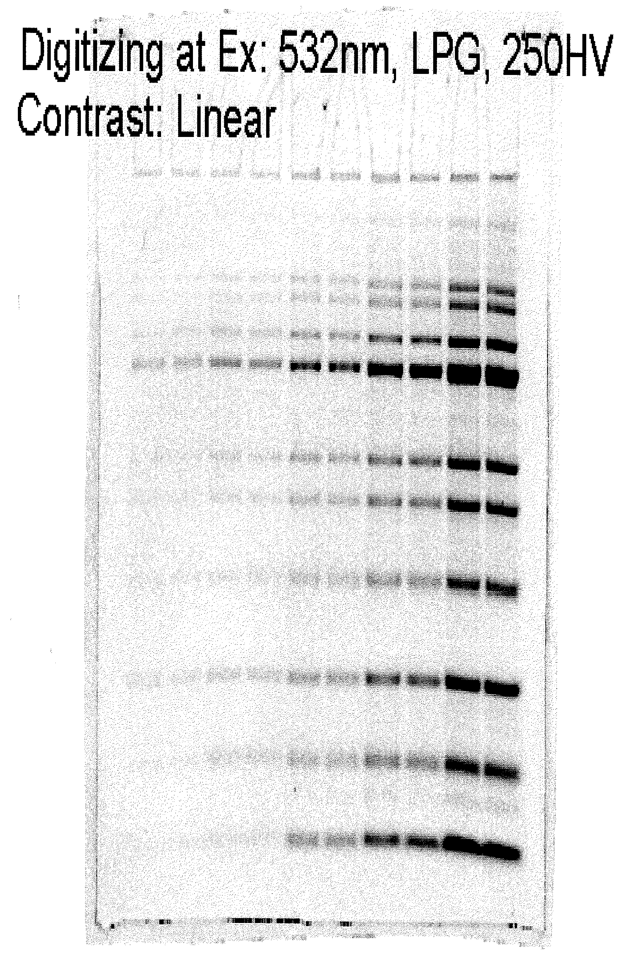

Figure 1 shows a digitized image of the gel stained with CBS that had been visually observed. Insulin existing at the lowest end is hardly seen in the lane with the lowest concentration.

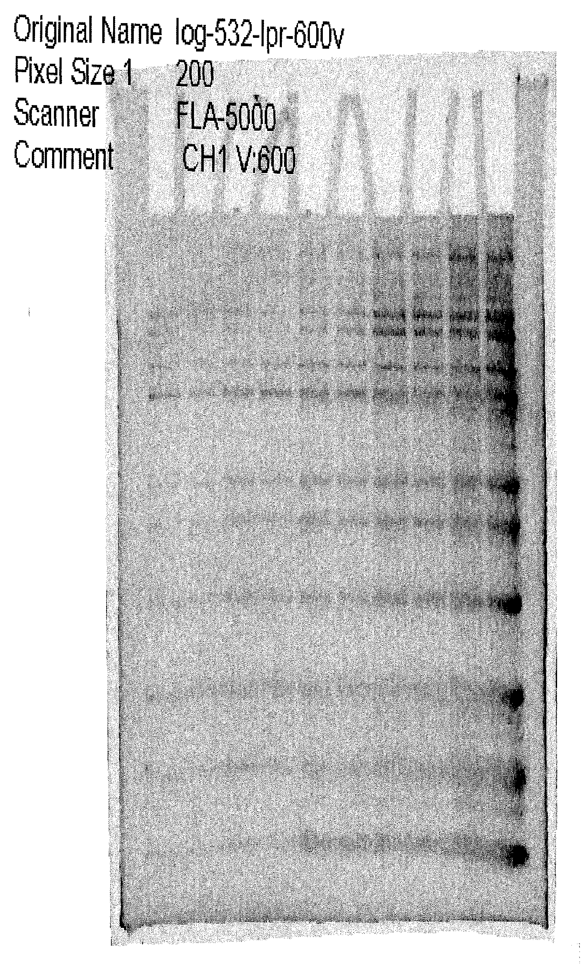

Figure 2 (comparative example) shows an image taken under conditions consisting of excitation at 532 nm, an LPG filter, 600 HV, Channel 1, and PMT. A great degree of noise may be observed throughout the entire image, and an insulin band with the lowest concentration may be noted to a slight degree.

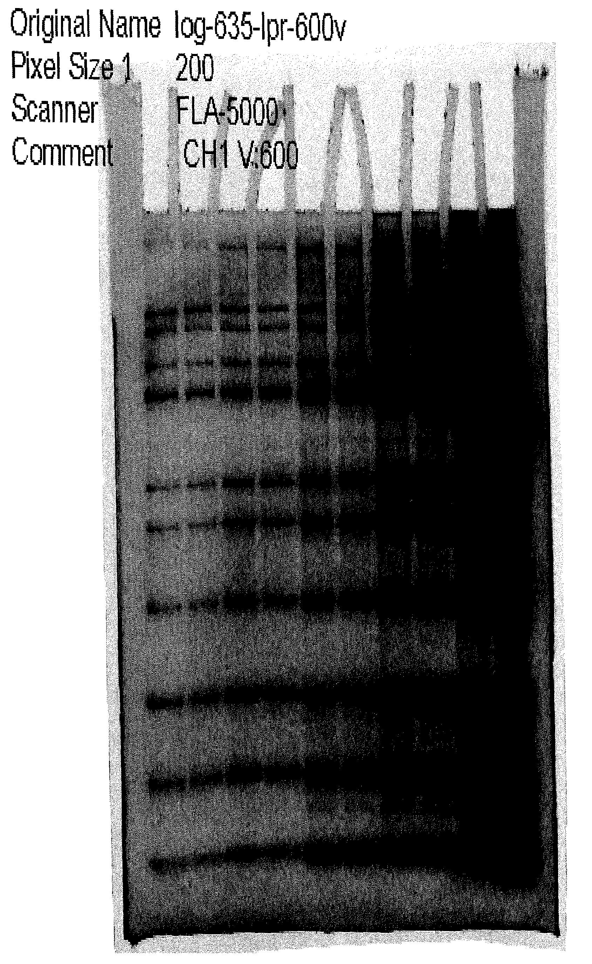

Figure 3 (the present invention) shows an image taken under conditions consisting of excitation at 635 nm, an LPR filter, 600 HV, Channel 1, and PMT. The background noise may be noted to a slight degree throughout the entire image, but the insulin band with the lowest concentration may be clearly seen.

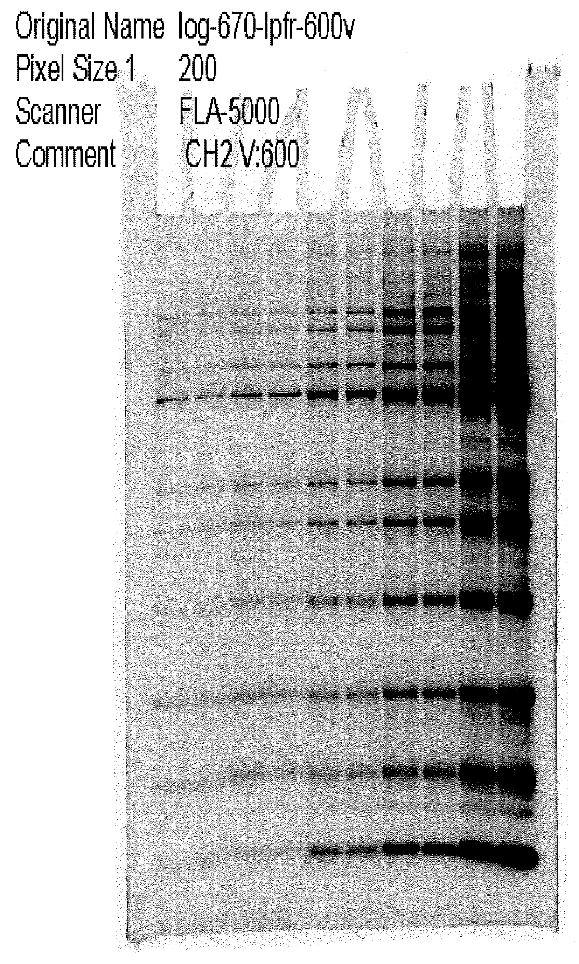

Figure 4 (the present invention) shows an image taken under conditions consisting of excitation at 670 nm, an LPFR filter, 600 HV, Channel 2, and PMT. The background noise of the entire image is lowest, and a smooth image can be obtained. The insulin band with the lowest concentration maybe clearly seen. Thus, this is the image that can be evaluated highest.

### Effects of the Invention

A protein is identified by cutting protein spots out of gel after completion of two-dimensional electrophoresis and then subjecting the resultant to mass spectroscopy. When SYPRO-Ruby, a highly sensitive fluorescence method, is used, since protein spots cannot visually be seen, a method of placing a paper that has been printed out to full scale underneath is adopted to confirm that proteins are cut from correct positions. However, since the gel itself is colorless and transparent, in order to confirm that proteins are cut from the correct positions, it is necessary to read out the image again after the operations.

According to the method of the present invention, since spots that are stained with CBB and can be visually observed are present together with spots that cannot be visually seen but can be detected at high sensitivity, it becomes possible to cut out protein spots that cannot be visually seen, while using as a clue the presence of peripheral spots that can be visually seen. Thus, according to the method of the present invention, it is possible to quickly and simply detect and analyze a trace amount of protein.

## Claims

1. A method for detecting a protein as a fluorescence image, which comprises staining a protein that has been electrophoresed on a gel with Coomassie Brilliant Blue and then applying excitation light having a wavelength of 600 nm or more to the protein.

2. The method according to claim 1 wherein excitation light having a wavelength of 600 nm or more is applied to a protein, and the generated fluorescence is photoelectrically detected, so as to generate a fluorescence image of the above protein.

3. The method according to claim 1 wherein excitation light having a wavelength between 600 nm and 700 nm is applied to the protein.

4. The method according to claim 1 wherein excitation light having a wavelength between 650 nm and 700 nm is applied to the protein.

5. The method according to claim 1 wherein the gel is a polyacrylamide gel.

6. The method according to claim 1 wherein after a protein has been stained with Coomassie Brilliant Blue, it is decolorized with a decolorizing solution, and excitation light having a wavelength of 600 nm or more is then applied to the resultant protein.

7. The method according to claim 1 wherein a protein existing on a gel is detected by visual observation, and at the same time, it is detected as a fluorescence image.
